# EUROPEAN PATENT APPLICATION

(11) **EP 3 178 479 A1**
(43) Date of publication of application: **14.06.2017**
(21) Application number: 16202742.9
(22) Date of filing: 07.12.2016
(51) Int. Cl.: A61K 31/4184, A61K 47/10, A61P 33/10, A61P 33/00

(54) **AN INJECTABLE COMPOSITION OF RICOBENDAZOLE**

(30) Priority: 08.12.2015 TR 201515599
(71) Applicant: Verano Ilac Sanayi Ve Ticaret A.S., Istanbul (TR)
(72) Inventor: TÜRKYILMAZ, Ali, 34460 Istanbul (TR); YELKEN, Gülay, 34460 Istanbul (TR); TOMBAYOGLU, Vildan, 34460 Istanbul (TR); PACALI, Mustafa, 34460 Istanbul (TR)
(74) Representative: Sevinç, Erkan

(57) **Abstract**

The present invention relates to an injectable composition comprising ricobendazole, lecithin and at least one pharmaceutically acceptable excipient.

## Description

### Field of Invention

The present invention relates to an injectable composition comprising ricobendazole, lecithin and at least one pharmaceutically acceptable excipient.

### Background of Invention

Antiparasitics are a class of medications which are indicated for the treatment of parasitic diseases such as those caused by endoparasites such as nematodes (roundworms), cestodes (tapeworms), trematodes (flatworms), and parasitic protozoa (the cause of malaria).

Anthelmintics are a group of antiparasitic drugs that expel parasitic worms (helminths) and other internal parasites from the body by either stunning or killing them and without causing significant damage to the host. They may also be called vermifuges (those that stun) or vermicides (those that kill). They are used to treat people or animals who are infected by helminths, a condition called helminthiasis.

Benzimidazole is a heterocyclic aromatic organic compound. This bicyclic compound consists of the fusion of benzene and imidazole.

Bezimidazoles are mostly used on livestock like cattle, sheep, goats, etc. Besides that, use on dogs and cats is available. It is available in the form of injectables and drenches or tablets, pills, etc.

Ricobendazole is a methylcarbamate benzimidazole with a broad-spectrum anthelmintic activity. Ricobendazole is a key metabolite of albendazole. Ricobendazole is a veteran anthelmintic (wormer) compound belonging to the chemical class of the benzimidazoles.

Chemical name of ricobendazole is Methyl [5-(propane-1-sulfinyl)-1 H-benzoimidazol-2-yl]-carbamate and structure is illustrated with Formula I given below.

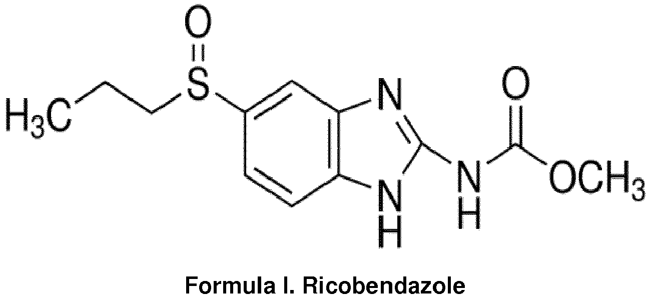

In prior art, albendazole derivatives and the use for the treatment of helminthiasis first disclosed in US3915986A. US4076828A discloses the parenteral administration of benzimidazole derivatives in treating helminthiasis. EP705101B1 discloses the oral administration of benzimidazole derivatives and the use as an anti-parasitic agent.

A problem found in the prior art with existing anthelmintic compounds including benzimidazole anthelmintics, is the need to deliver the compound by injection due to the particularly poor stability. In addition, poor solubility of this type of compound in aqueous environments. As the solubility of the anthelmintic is often poor, injections are not readily absorbed and often may cause pain to the animal or human patient due to inadequate absorption or precipitate formation.

Another problem in the prior art, during the production process water soluble and oil-soluble ingredients are generally dissolved in the aqueous phase and oil phase, respectively. Emulsifiers, such as phosphatides, can be dispersed in either oil or aqueous phase. Both phases are adequately heated and stirred to disperse or dissolve the ingredients. The lipid phase is then generally added to the aqueous phase under controlled temperature and agitation (using high-shear mixers) to form a homogenously dispersed coarse formulation. But heating during the process can have negative effect on stability.

A further problem noted with prior art formulations is pain, swelling and inflammation at the injection site. These reactions may also be associated with the anthelmintic compound such as a reaction from the subject against precipitated drug.

In this invention, to fulfill requirements of the injectable composition of benzimidazoles, a stable injectable composition have been developed comprising ricobendazole.

### Description of Invention

The present invention relates to the injectable composition comprises;
- ricobendazole
- lecithin
- at least one pharmaceutically acceptable excipient.

Benzimidazoles are characterised by having poor stability and poor solubility/dispersion characteristics in an aqueous environment. It is understood by the inventors that this poor solubility/dispersion may result in corresponding poor absorption, which may lead to poor pharmacokinetics. If such a compound is administered absent of a suitably formulated delivery system, the compound may either not be absorbed or be only poorly absorbed within an aqueous environment such as the blood stream.

The main object of the present invention is to provide a stable injectable composition of a ricobendazole.

Another object of the present invention is to enhance solubility of ricobendazole in aqueous environments.

According to a further aspect of the present invention there is provided the use of a formulation substantially as described above in the manufacture of a medicament to increase the stability and solubility/dispersion of ricobendazole wherein ricobendazole is characterized by having poor stability and solubility/dispersion characteristics in an aqueous environment.

According to an embodiment of the present invention, the injectable composition contains ricobendazole in an amount of 90 mg to 110 mg of 1 mL of the pharmaceutical formulation.

According to this embodiment, the injectable composition comprising ricobendazole is administered by intravenous, subcutaneously, intramuscular, intradermal, intracutaneous, intraruminal.

Ricobendazole is the injectable benzimidazole. Chemically ricobendazole is albendazole sulfoxide, the major metabolite of albendazole. Benzimidazoles are very poorly soluble in water, and therefore cannot be injected, because they get stuck at the injection site. They have to be delivered orally (in drenches, boluses, tablets, etc.). Ricobendazole is significantly more soluble in water than albendazole and can be injected.

Ricobendazole is an anti-parasitic nitroimidazole compound administered by injection, unlike most nitroimidazoles. Ricobendazole binds the colchicine site of tubulin, inhibiting microtubule polymerization. Ricobendazole also exhibits anticancer activity, potentiating the effects of taxanes and inhibiting cell proliferation in breast cancer cells, non-small cell lung cancer (NSCLC) cells, and melanoma cells.

The injectable composition comprising ricobendazole of this invention comprises one or more pharmaceutically acceptable excipient which is selected from solvents or mixtures thereof.

Natural lecithin, obtained from egg yolk, has been used extensively to stabilize the injectable formulation. This dispersing agent is biocompatible, nontoxic and is metabolized like natural fat. In this present invention, it has been found that, the degree of dispersion and stability of the composition can be optimized by selecting commercial lecithin containing an adequate fraction of negatively charged phospholipids.

According to an embodiment of the present invention, the injectable composition contains lecithin in an amount of 10 mg to 40 mg of 1 mL of the pharmaceutical formulation.

According to this embodiment of the present invention, the injectable composition contains ricobendazole and lecithin in a special ratio which is the weight ratio of lecithin to ricobendazole is between 0.1 to 1.0, preferably 0.1 to 0.5 and more preferably 0.2 (w/w).

In one embodiment, the injectable composition comprising solvent is selected from corn oil (maize), cottonseed oil, dibutyl phthalate, diethyl phthalate, dimethyl ether, dimethyl phthalate, dimethyl sulfoxide, dimethylacetamide, ethyl acetate, ethyl alcohol (ethanol), ethyl lactate, ethyl oleate, glycerin, glycofurol, isopropyl alcohol, isopropyl myristate, isopropyl palmitate, light mineral oil, medium-chain triglycerides, methyl lactate, mineral oil, monoethanolamine, monopropylene glycol, octyldodecanol, olive oil, peanut oil, polyethylene glycol, polyoxyl 35 castor oil, propylene carbonate, propylene glycol, pyrrolidone, safflower oil, sesame oil, soybean oil, sunflower oil, triacetin, tricaprylin, triethanolamine, triethyl citrate, triolein, water, water-miscible or mixtures thereof.

In a preferred embodiment, the solvents are preferably ethyl alcohol (ethanol) and monopropylene glycol.

In one embodiment, the injectable composition comprises;
- ricobendazole
- lecithin
- ethyl alcohol (ethanol)
- monopropylene glycol.

In one embodiment, the injectable composition is used as anti-parasitic, anti-helmintic, anti-trematode, taenicide, nematicide.

In another embodiment, the lyophilized injectable composition is formulated for administration to human or animal.

Ricobendazole is used therapeutically in the treatment of helminthiasis and tapeworm infestations in man and animal. It prevents growth of parasites; reduce parasite numbers; kill parasites; kill incoming parasite larvae; lower the amount of incoming parasite larva and combinations thereof.

Ricobendazole is effective against gastrointestinal roundworms and lungworms of livestock including adults and L4-larvae of the most important species (e.g. of the genus *Bunostomum, Haemonchus, Ostertagia, Teladorsagia, Trichostrongylus, Cooperia, Nematodirus, Chabertia, Oesophagostomum, Trichuris, Dictyocaulus,* etc.) as well as arrested larvae of several species. It is also effective against most livestock tapeworms (e.g. *Moniezia*) and against adult liver flukes (*Fasciola hepatica* and *Fascioloides magna*)*.* It is also effective against the major parasitic roundworms of dogs and cats (e.g*.Ancylostoma, Toxocara, Trichuris, Uncinaria*).

Formulation components and process parameters play critical role in the success of injectable compositions as drug delivery vehicles and hence need to be well integrated in the formulation development strategies. Physico-chemical properties of active therapeutic agents significantly impact pharmacokinetics and tissue disposition following injectable administration and hence need special attention while selecting such delivery vehicles.

There are also a number of challenges surrounding the formulation and development of injectable forms such as stability and solubility. Moreover, active pharmaceutical agents in liquid forms are more susceptible to chemical and physical instability than the solid state. In addition to the solubility, ricobendazole needs to be physically and chemically stable in the injectable solution. Trace amounts of impurities from active pharmaceutical agents or excipients and the pH of the solution may cause the degradation of active pharmaceutical agent and this may cause an increase in instability when the solution is consistently introduced into atmosphere.

The chemical stability of ricobendazole has been provided throughout the shelf life by selecting optimum excipients like lecithin with a specific amount. In addition, with cold process, precipitation and crystallization in the solution has been prevented.

According to an embodiment of the present invention, said injectable composition comprising ricobendazole comprises;
- ricobendazole in an amount of 90 mg to 110 mg of 1 mL of the pharmaceutical formulation
- lecithin in an amount of 10 mg to 40 mg of 1 mL of the pharmaceutical formulation.
- ethyl alcohol (ethanol)
- monopropylene glycol

The process for the preparation of the injectable composition comprising ricobendazole comprises the following steps:
I. ricobendazole and monopropylene glycol are loaded in the product tank and mixed for a while.
II. lecithin and ethyl alcohol (ethanol) are loaded in the other product tank and mix for a while and loaded the first product tank and mix again.
III. the mixture is then filtered and filled in the bottle/vial or filled in the bottle/vial by performing sterilization such as gamma, autoclave etc.

It has been found that, lecithin and cold process enhances physical and chemical stability of ricobendazole in the present injectable composition. The pharmaceutical formulation disclosed herein is a stable solution wherein ricobendazole or pharmaceutically acceptable excipients are not precipitated.

### Example 1:

| **Ingredients** | **Amount in unit formula mg/mL** |
|---|---|
| Ricobendazole | 100 |
| Ethanol (absolute) | 158 |
| Lecithin | 20 |
| Monopropylene gylcol | q.s. 1 mL |

| | |
|---|---|
| *q.s.:quantity sufficient* | |

**Production process:** Monopropylene glycol and ricobendazole are loaded in the product tank and mixed for a while. Ethanol and lecithin are loaded in the other product tank and mix for a while and loaded the first product tank and mix again. The mixture is then filtered and filled in the bottle/vial or filled in the bottle/vial by performing sterilization such as gamma, autoclave etc.

## Claims

1. The injectable composition, wherein the composition comprising;
• ricobendazole
• lecithin
• at least one pharmaceutically acceptable excipient.

2. The injectable composition according to claim 1, wherein ricobendazole in an amount of 90 mg to 110 mg of 1 mL of the pharmaceutical formulation.

3. The injectable composition according to claim 1, wherein the composition is administered by intravenous, subcutaneously, intramuscular, intradermal, intracutaneous, intraruminal.

4. The injectable composition according to claim 1, wherein the composition comprising one or more pharmaceutically acceptable excipient which is selected from solvents or mixtures thereof.

5. The injectable composition according to claim 1, wherein lecithin in an amount of 10 mg to 40 mg of 1 mL of the pharmaceutical formulation.

6. The injectable composition according to claim 1 or 5, wherein the weight ratio of lecithin to ricobendazole is between 0.1 to 1.0, preferably 0.1 to 0.5 and more preferably 0.2 (w/w).

7. The injectable composition according to claim 4, wherein solvent is selected from corn oil (maize), cottonseed oil, dibutyl phthalate, diethyl phthalate, dimethyl ether, dimethyl phthalate, dimethyl sulfoxide, dimethylacetamide, ethyl acetate, ethyl alcohol (ethanol), ethyl lactate, ethyl oleate, glycerin, glycofurol, isopropyl alcohol, isopropyl myristate, isopropyl palmitate, light mineral oil, medium-chain triglycerides, methyl lactate, mineral oil, monoethanolamine, monopropylene glycol, octyldodecanol, olive oil, peanut oil, polyethylene glycol, polyoxyl 35 castor oil, propylene carbonate, propylene glycol, pyrrolidone, safflower oil, sesame oil, soybean oil, sunflower oil, triacetin, tricaprylin, triethanolamine, triethyl citrate, triolein, water, water-miscible or mixtures thereof.

8. The injectable composition according to claim 7, wherein solvents are preferably ethyl alcohol and monopropylene glycol.

9. The injectable composition according to any preceding claim, wherein the composition comprising;
• ricobendazole
• lecithin
• ethyl alcohol
• monopropylene glycol.

10. The injectable composition according to any preceding claim, wherein the composition is used as anti-parasitic, anti-helmintic, anti-trematode, taenicide, nematicide.

11. The injectable composition to any preceding claim, wherein the composition is formulated for administration to human or animal.

12. The injectable composition according to any preceding claim, wherein the composition comprising;
• ricobendazole in an amount of 90 mg to 110 mg of 1 mL of the pharmaceutical formulation
• lecithin in an amount of 10 mg to 40 mg of 1 mL of the pharmaceutical formulation
• ethyl alcohol
• monopropylene glycol.
